(19) **Europäisches Patentamt**
**European Patent Office**
**Office européen des brevets**

(11) **EP 4 107 522 B1**

(12) **EUROPÄISCHE PATENTSCHRIFT**

(45) Veröffentlichungstag und Bekanntmachung des Hinweises auf die Patenterteilung:
**02.07.2025 Patentblatt 2025/27**

(21) Anmeldenummer: **21728445.4**

(22) Anmeldetag: **06.05.2021**

(51) Internationale Patentklassifikation (IPC):
**G01N 33/00** (2006.01)  **G06N 3/08** (2023.01)

(52) Gemeinsame Patentklassifikation (CPC):
**G01N 33/0034; G06N 3/08**

(86) Internationale Anmeldenummer:
**PCT/EP2021/061978**

(87) Internationale Veröffentlichungsnummer:
**WO 2021/228675 (18.11.2021 Gazette 2021/46)**

(54) **COMPUTERGESTÜTZTES VERFAHREN ZUM ERZEUGEN VON TRAININGSDATEN FÜR EIN NEURONALES NETZ FÜR EINE VORHERSAGE EINER SCHADSTOFFKONZENTRATION AN EINER MESSSTATION**

COMPUTER-ASSISTED METHOD FOR GENERATING TRAINING DATA FOR A NEURAL NETWORK FOR PREDICTING A CONCENTRATION OF POLLUTANTS AT A MEASURING STATION

PROCÉDÉ ASSISTÉ PAR ORDINATEUR POUR GÉNÉRER DES DONNÉES D'APPRENTISSAGE POUR UN RÉSEAU NEURONAL DESTINÉ À PRÉDIRE UNE CONCENTRATION DE POLLUANTS AU NIVEAU D'UNE STATION DE MESURE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.05.2020 DE 102020206047**

(43) Veröffentlichungstag der Anmeldung:
**28.12.2022 Patentblatt 2022/52**

(73) Patentinhaber: **Siemens Aktiengesellschaft**
**80333 München (DE)**

(72) Erfinder:
• **JAEGER, Florian Ansgar**
**10961 Berlin (DE)**
• **MÜLLER, Katrin**
**10713 Berlin (DE)**

(74) Vertreter: **Siemens Patent Attorneys**
**Postfach 22 16 34**
**80506 München (DE)**

(56) Entgegenhaltungen:
**EP-A1- 3 627 403**

• **ZHENG YU YUZHENG@MICROSOFT COM ET AL:**
**"Forecasting Fine-Grained Air Quality Based on Big Data", HOT TOPICS IN MIDDLEBOXES AND NETWORK FUNCTION VIRTUALIZATION, ACM, 2 PENN PLAZA, SUITE 701 NEW YORK NY 10121-0701 USA, 10 August 2015 (2015-08-10), pages 2267 - 2276, XP058513868, ISBN: 978-1-4503-3540-9, DOI: 10.1145/ 2783258.2788573**

**Beschreibung**

Die Erfindung betrifft ein Verfahren zum Erzeugen von Trainingsdaten für ein neuronales Netz gemäß dem Oberbegriff des Patentanspruches 1, ein Verfahren zum Training eines neuronalen Netzes gemäß dem Oberbegriff des Patentanspruches 6 sowie ein Verfahren zum Ermitteln einer Schadstoffkonzentration mittels eines neuronalen Netzes gemäß dem Oberbegriff des Patentanspruches 7.

Die Schadstoffbelastung, beispielsweise eine Stickoxidkonzentration, kann innerhalb einiger deutscher Städte für bestimmte Zeiträume oberhalb der zulässigen Grenzwerte liegen. Zur Sicherstellung einer ausreichenden Luftqualität können Städte mehrere Maßnahmen treffen, beispielsweise Fahrverbote. Allerdings ist es für die Wirksamkeit dieser Maßnahmen erforderlich, dass diese bereits vor einem möglichen Überschreiten der Grenzwerte durchgeführt werden. Hierzu ist eine zuverlässige und möglichst präzise Vorhersage (Prognose) der Schadstoffkonzentration erforderlich.

Grundsätzlich werden Emissionen sowie Immissionen (Dimension Masse oder Masse pro Länge pro Zeit) und Konzentrationen (Dimension Masse pro Volumen) unterschieden. Die Emission ist die emittierte Masse eines Schadstoffes, beispielsweise eines Verkehrsteilnehmers innerhalb eines Zeitbereiches, beispielsweise einer Stunde. Die Emission kann ebenfalls auf eine Länge (Straßenlänge, Streckenlänge usw.) und einen Zeitbereich bezogen sein, sodass diese hierbei die Dimension Masse pro Länge pro Zeit aufweist. Die Schadstoffkonzentration wird, beispielsweise von einer Messstation, an einem bestimmten Ort innerhalb der Stadt, basierend auf die dortige Immission gemessen. Grundsätzlich sind die Emissionen, Immissionen und Schadstoffkonzentrationen zeitabhängig.

Die Schadstoffkonzentration ist aufgrund der Komplexität der Vorgänge schwer vorherzusagen, sodass hierfür typischerweise neuronale Netze verwendet werden.

Ein solches Vorhersageverfahren ist beispielsweise beschrieben in Zheng, Yu et al: "Forecasting Fine-Grained Air Quality Based on Big Data" , 10. August 2015, Seiten 2267-2276, XP058513868, DOI: 10.1145/2783258.2788573.

Das grundsätzliche Verfahren ist hierbei zweigeteilt. Zunächst wird die Emission mittels eines Modells berechnet. Anschließend wird die Schadstoffkonzentration aus der modellbasiert berechneten Emission mittels des neuronalen Netzes ermittelt.

Hierzu ist ein Training des neuronalen Netzes erforderlich, das heißt es sind Trainingsdaten bezüglich der Schadstoffkonzentration erforderlich. Sinnbildlich muss das neuronale Netz mittels der Trainingsdaten lernen, wie sich die Schadstoffkonzentration aus der Schadstoffemission ergibt. Typischerweise werden zum Trainieren des neuronalen Netzes historische Daten der Schadstoffkonzentration als Trainingsdaten verwendet. Ein derart trainiertes neuronales Netz liefert in Situationen, die häufig auftreten, eine gute Vorhersage. Daher kann die durchschnittliche Schadstoffkonzentration durch diese ausreichend genau vorhergesagt werden.

Problematisch sind Ereignisse oder Situationen hoher Belastung, da diese typischerweise selten sind. Dadurch stehen nur wenig Daten zum Training des neuronalen Netzes zur Verfügung. Durch dieses Problem ist die Vorhersage für die eigentlich interessanten Ereignisse hoher Belastung, das heißt für die seltenen Ereignisse, schlechter.

Aus dem Stand der Technik sind im Wesentlichen zwei Verfahren zur Verbesserung der Vorhersage bezüglich solcher seltenen Ereignisse bekannt.

Erstens können die für das Training verwendeten Daten beziehungsweise Messreihen unterschiedlich gewichtet werden. Beispielsweise wird ein historisches Ereignis hoher Belastung mehrmals verwendet. Nachteilig hieran ist, dass dadurch die Vorhersage der durchschnittlichen Belastung verschlechtert wird. Somit bleibt das eigentliche Problem, dass für Ereignisse hoher Belastung weniger Messreihen beziehungsweise Messdaten und somit Trainingsdaten vorliegen, bestehen. Zweitens können die Schadstoffemission und Schadstoffkonzentration durch einen vollständigen modellbasierten Ansatz berechnet werden. Dies ist ein großer Aufwand und zudem sind nicht alle Abhängigkeiten bekannt. Die bekannten Verfahren liefern daher typischerweise zu geringe Werte für die Schadstoffkonzentration.

Der vorliegenden Erfindung liegt die Aufgabe zugrunde, ein verbessertes Training eines neuronalen Netzes, welches zur Ermittlung einer Schadstoffkonzentration aus einer Schadstoffemission vorgesehen ist, bereitzustellen.

Die Aufgabe wird durch ein Verfahren zum Erzeugen von Trainingsdaten für ein neuronales Netz mit den Merkmalen des unabhängigen Patentanspruches 1, durch ein Verfahren zum Training eines neuronalen Netzes mit den Merkmalen des unabhängigen Patentanspruches 6, sowie durch ein Verfahren zum Ermitteln einer Schadstoffkonzentration mit den Merkmalen des unabhängigen Patentanspruches 7 gelöst. In den abhängigen Patentansprüchen sind vorteilhafte Ausgestaltungen und Weiterbildungen der Erfindung angegeben.

Das erfindungsgemäße Verfahren zum Erzeugen von Trainingsdaten stellt Daten beziehungsweise eine Zeitreihe der Schadstoffkonzentration bereit, mittels welchem das neuronale Netz trainiert werden kann. Das Training kann mittels bekannter Verfahren, beispielsweise Deep-Learning, erfolgen.

Das neuronale Netz (englisch: Artificial Neural Network) ist hierbei dazu vorgesehen oder dazu ausgebildet, aus einer Schadstoffemission eine Schadstoffkonzentration an der Messstation zu ermitteln. Die Schadstoffimmission beziehungsweise die Schadstoffimmissionen werden mittels des Transmissionsmodells aus der Schadstoffemission beziehungsweise Schadstoffemissionen berechnet. Das Transmissionsmodell modelliert somit den Transport der emittierten Schadstoffe vom Ort der Emission, beispielsweise einer Straße, zum Ort der Immission, das heißt zur Messstelle. Das Transmissionsmodell kann bevorzugt chemische Umwandlungsprozesse und zugehörige Gleichungen umfassen.

Im ersten Schritt des erfindungsgemäßen Verfahrens zum Erzeugen der Trainingsdaten wird eine Messreihe einer Schadstoffkonzentration bereitgestellt, wobei wenigstens ein Wert beziehungsweise Messwert der Schadstoffkonzentration oberhalb des festgelegten Schwellenwertes ist. Mit anderen Worten wird eine Messreihe bereitgestellt, die zu einer wenigstens zu einem Zeitpunkt hohen Schadstoffkonzentration und somit zu einer hohen Schadstoffbelastung korrespondiert. Somit lag ein seltenes Ereignis einer hohen Schadstoffbelastung vor.

Der Schwellenwert ist typischerweise durch einen Grenzwert festgelegt, beispielsweise 200 Mikrogramm pro Kubikmeter ($\mu$g/m$^3$) für Stickoxid. Die Messreihe ist eine zeitliche Abfolge (kontinuierlich oder diskret) von Messwerten der Schadstoffkonzentration, beispielsweise in der Einheit $\mu$g/m$^3$. Die Messreihe weist einen oder mehrere Messwerte auf, wobei jeder Messwert zu einem bestimmten Zeitpunkt erfasst worden ist. Der Zeitpunkt kann ebenfalls ein Zeitbereich sein, sodass für den Zeitbereich ein Messwert erfasst oder ermittelt wurde. Beispielsweise wird für jede Stunde ein Messwert der Schadstoffkonzentration ermittelt, beispielsweise durch eine oder mehrere Messungen. Mit anderen Worten wird beispielsweise für jede Stunde eines Tages ein Messwert der Schadstoffkonzentration erfasst. Die zeitliche geordnete Abfolge dieser erfassten Messwerte bildet dann eine exemplarische Messreihe der Schadstoffkonzentration aus.

Im zweiten Schritt des erfindungsgemäßen Verfahrens zum Erzeugen der Trainingsdaten wird wenigstens eine Messreihe einer physikalischen/technischen Messgröße bereitgestellt. Hierbei ist die Messgröße eine physikalische/technische Grö-$\beta$e, beispielsweise eine Temperatur, eine Windgeschwindigkeit und/oder eine Windrichtung und/oder eine oder mehrere chemische Stoffkonzentrationen. Die Messgröße ist zur bereitgestellten gemessenen Schadstoffkonzentration zugehörig, das heißt, dass für jeden Zeitpunkt ein Messwert der Schadstoffkonzentration und ein Messwert der Messgröße vorliegt. Mehrere Messgrößen und entsprechende Messreihen können vorgesehen sein.

Beispielsweise wird für jede Stunde eines Tages eine durchschnittliche Schadstoffkonzentration und die bei der jeweiligen durchschnittlichen Schadstoffkonzentration vorliegende und somit zugehörige durchschnittliche Temperatur, Windgeschwindigkeit und/oder Windrichtung erfasst. Mit anderen Worten werden wenigstens zwei Messgrößen zeitlich erfasst, die Schadstoffkonzentration und die physikalische/technische Messgröße, beispielsweise die Temperatur, die Windgeschwindigkeit und/oder die Windrichtung, die bei der gemessenen Schadstoffkonzentration vorliegen beziehungsweise vorlagen. Die Messgröße ist deshalb von Bedeutung, da diese oder mehrere Messgrößen, wie beispielsweise die Temperatur, die Windgeschwindigkeit und/oder Windrichtung und/oder eine chemische Zusammensetzung der Luft (chemische Stoffkonzentrationen), die Schadstoffkonzentration grundsätzlich beeinflussen, das heißt die Schadstoffkonzentration ist von der einen oder mehreren Messgrößen abhängig. So kann die Schadstoffkonzentration an der Messstation innerhalb einer Stadt entscheidend von der Windrichtung und/oder der Windgeschwindigkeit sowie der chemischen Zusammensetzung der Luft abhängen.

Im dritten Schritt des erfindungsgemäßen Verfahrens zum Erzeugen der Trainingsdaten wird das Transmissionsmodell bereitgestellt, wobei das Transmissionsmodell einen Zusammenhang (Abhängigkeit) zwischen der Schadstoffemission, der Messgröße und der Schadstoffimmission an der Messstation modelliert beziehungsweise beschreibt. Mittels des Transmissionsmodells kann somit die Schadstoffimmission, beispielsweise von Verkehrsteilnehmern, in Abhängigkeit der Messgröße, beispielsweise der Temperatur, der Windgeschwindigkeit und/oder der Windrichtung und/oder chemischen Stoffkonzentrationen, berechnet werden. Typischerweise sind diese Transmissionsmodelle komplex und umfassen ergänzend Gleichungen bezüglich chemischer Umwandlungsprozesse innerhalb der Luft. Das Transmissionsmodell weist somit Eingangsgrößen und wenigstens eine Ausgangsgröße auf, wobei die Eingangsgrößen die Schadstoffemission und Messgröße sind, und die Ausgangsgrößen die Schadstoffimmission an der Messstation ist.

Im vierten Schritt des erfindungsgemäßen Verfahrens zum Erzeugen der Trainingsdaten wird ein erster Wert $I_0$ der Schadstoffimmission mittels des Transmissionsmodells aus der Schadstoffemission berechnet. Hierzu wird wenigstens eine zu einem Wert $C_0$ der bereitgestellten gemessenen Schadstoffkonzentration zugehöriger Messwert der Messgröße verwendet. Mit anderen Worten wird der zum Wert $C_0$ der bereitgestellten gemessenen Schadstoffkonzentration zugehörige Wert der Messgrö-$\beta$e, beispielsweise der zum Wert der Schadstoffkonzentration zugehörige Wert der Temperatur, als eine Eingangsgröße des Modells verwendet. Die weitere Eingangsgröße ist die Schadstoffemission, die ebenfalls berechnet werden kann. Hieraus berechnet das Transmissionsmodell dann den ersten Wert $I_0$ der Schadstoffimmission. Beispielsweise werden Temperatur, Windgeschwindigkeit und/oder Windrichtung in das Transmissionsmodell als Eingangsgrößen gegeben, woraus das Transmissionsmodell dann die erste Schadstoffimmission $I_0$ an der Messstation beziehungsweise in einem Bereich der Messstation berechnet.

Im fünften Schritt des erfindungsgemäßen Verfahrens zum Erzeugen der Trainingsdaten wird ein zweiter Wert $I_1$ der Schadstoffimmission mittels des Transmissionsmodells basierend auf denselben Schadstoffemissionen berechnet. Die Schadstoffemissionen bleiben somit unverändert. Allerdings wird der für das Berechnen des ersten Wertes $I_0$ der Schadstoffimmission verwendete Messwert der Messgröße numerisch verändert. Mit anderen Worten wird die zweite Schadstoffimmission $I_1$ für einen veränderten Wert der Messgröße, beispielsweise für einen veränderten Wert der Temperatur, der Windgeschwindigkeit und/oder der Windrichtung und/oder veränderte Stoffkonzentrationen von chemischen Substanzen in der Luft, beispielsweise Stickoxide, Sauerstoff und/oder Ozon, berechnet. Der veränderte Wert der Messgröße oder dementsprechend die veränderte Messreihe der Messgröße wird somit als Eingangsgröße in das Transmissionsmodell gegeben. Dadurch wird der zweite Wert der Schadstoffimmission $I_1$ beziehungsweise eine zweite Schadstoffimmission beziehungsweise eine zweite Zeitreihe der Schadstoffimmission berechnet. In diesem Sinne korrespondiert der zweite Wert der Schadstoffimmission $I_1$ zu einer synthetischen Schadstoffimmission, die beim entsprechenden veränderten Wert der Messgröße, beispielsweise bei einer veränderten Temperatur, einer veränderten Windgeschwindigkeit und/oder einer veränderten Windrichtung und/oder veränderten chemischen Randbedingungen vorliegen würde. Hierbei ist es vorteilhaft den Wert der Messgröße nur geringfügig zu verändern. Beispielsweise ist die relative Änderung des Wertes der Messgröße bevorzugt kleiner als 10 Prozent.

Im sechsten Schritt des erfindungsgemäßen Verfahrens wird eine neue, weitere oder synthetische Messreihe erzeugt, die dem Trainingsdatensatz zugrunde liegt. Mit anderen Worten umfasst der Trainingsdatensatz die neue Messreihe, wobei das neuronale Netz mittels der neuen Messreihe trainierbar ist beziehungsweise trainiert wird oder wurde. Die neue Messreihe wird durch eine Veränderung $\Delta C$ des Wertes $C_0$ der bereitgestellten gemessenen Messreihe der Schadstoffkonzentrationen erzeugt, wobei die Veränderung $\Delta C$ mittels der relativen Änderung $\Delta I/E_0 = (I_1 - I_0)/I_0$ der berechneten Werte der Schadstoffimmissionen erfolgt. Hierbei sind alle zur genannten relativen Änderung mathematisch äquivalenten Gleichungen ebenfalls umfasst. Da der neuen Messreihe der zweite (synthetisch) berechnete Wert $I_1$ der Schadstoffimmission zugrunde liegt, und der zweite Wert $I_1$ eben nicht auf einen gemessenen Wert der Messgröße basiert, kann die neue beziehungsweise weitere Messreihe der Schadstoffkonzentration ebenfalls als synthetische Messreihe bezeichnet werden. Mit anderen Worten ist die neu erzeugte Messreihe im Hinblick auf die bereitgestellte gemessene Messreihe nicht gemessen worden, sondern mittels des beschriebenen Verfahrens synthetisch erzeugt.

Durch die vorliegende Erfindung kann somit eine Mehrzahl von synthetischen Messreihen der Schadstoffkonzentration erzeugt werden, mit welchem das neuronale Netz, wie bereits mit der ursprünglich gemessenen Messreihe der Schadstoffkonzentration, trainiert werden kann. Da die ursprüngliche bereitgestellte gemessene Messreihe der Schadstoffkonzentration und somit auch die zugehörigen Messwerte der Messgröße und die Schadstoffemission zu einem selten Ereignis hoher Belastung korrespondiert beziehungsweise führten - was durch den Schwellenwert des ersten Schrittes des vorliegenden Verfahrens sichergestellt ist, können somit mehrere Messreihen von seltenen Ereignissen hoher Belastung synthetisch erzeugt werden. Das ist deshalb der Fall, da der Messwert der Messgröße erfindungsgemäß ausgehend vom tatsächlichen beim Auftreten des seltenen Ereignisses gemessenen Messwert verändert wird. Wird das neuronale Netz mittels dieser neu erzeugten synthetischen Messreihen trainiert, so wird die Vorhersage des neuronalen Netzes bezüglich der genannten seltenen Ereignisse verbessert, ohne das eine Verschlechterung des durchschnittlichen Verhaltens zu erwarten ist. Dadurch kann die Schadstoffkonzentration bezüglich hoher Werte verbessert vorhergesagt werden.

Mit anderen Worten ermöglicht die vorliegende Erfindung es dem neuronalen Netz von einem umfangreicheren Trainingsdatensatz zu lernen. Dadurch wird die Vorhersage des neuronalen Netzes bezüglich der seltenen, aber relevantesten Ereignisse hoher Belastung verbessert.

Weiterhin ist die Integration des Vorhersagealgorithmus in bereits bestehende Modelle nicht komplexer als die Verwendung herkömmlicher Algorithmen für neuronale Netze. Das ist deshalb der Fall, da diese zwar verbessert werden, aber in ihrer Struktur unverändert bleiben. Mit anderen Worten betrifft die vorliegende Erfindung zunächst das Trainieren des neuronalen Netzes beziehungsweise das Erzeugen eines zugehörigen Trainingsdatensatzes beziehungsweise ein Erweitern eines bereits bestehenden Trainingsdatensatzes.

Im Vergleich zu einer Gewichtung von Messwerten kann ebenfalls eine wesentlich bessere Datenbasis erzeugt werden. Verglichen mit einem vollständigen modellbasierten Ansatz sind Aufwand und Datenanforderung deutlich geringer. Weiterhin muss das Transmissionsmodell nicht online für eine Vorhersage betrieben werden, sondern muss lediglich für die spezifischen und relevanten Ereignisse oder Szenarien zum Trainieren des neuronalen Netzes laufen. Dadurch kann vorteilhafterweise Rechenzeit eingespart werden. Ein Onlinebetrieb kann jedoch vorgesehen sein.

Die vorliegende Erfindung ermöglicht somit eine genauere Vorhersage bei einem geringeren Aufwand und reduzierten Datenanforderungen.

Das erfindungsgemäße computergestützte Verfahren zum Trainieren eines neuronalen Netzes, wobei das neuronale Netz zum Ermitteln einer Schadstoffkonzentration aus wenigstens einer Schadstoffemission ausgebildet ist, ist gekennzeichnet dadurch, dass zum Trainieren des neuronalen Netzes ein gemäß der vorliegenden Erfindung und/oder einer ihrer Ausgestaltungen erzeugter Trainingsdatensatz verwendet wird.

Es ergeben sich zum erfindungsgemäßen Verfahren zum Erzeugen der Trainingsdaten gleichartige und gleichwertige Vorteile und Ausgestaltungen.

Das erfindungsgemäße computergestützte Verfahren zum Ermitteln einer Schadstoffkonzentration mittels eines neuronalen Netzes und mittels eines Domain-Modells der Schadstoffemission, wobei das neuronale Netz zum Ermitteln einer Schadstoffkonzentration aus der Schadstoffemission ausgebildet und gemäß der vorliegenden Erfindung und/oder einer ihrer Ausgestaltungen trainiert ist, wobei das Domain-Modell einen Zusammenhang zwischen einer physikalischen/technischen Messgrö-βe, insbesondere einer Temperatur, einer Windgeschwindigkeit und/oder einer Verkehrsstärke, und der Schadstoffemission modelliert, ist gekennzeichnet durch folgende Schritte:

- Berechnen eines Wertes der Schadstoffemission mittels des Domain-Modells, wobei hierzu wenigstens ein Messwert der Messgröße verwendet wird; und
- Ermitteln der Schadstoffkonzentration aus dem berechneten Wert der Schadstoffemission mittels des neuronalen Netzes.

Dadurch wird vorteilhafterweise eine Vorhersage für die Schadstoffkonzentration bereitgestellt. Die Vorhersage entspricht der ermittelten Schadstoffkonzentration. Basierend auf der ermittelten Schadstoffkonzentration können technische Maßnahmen vorgesehen sein, die zu einer tatsächlichen Reduzierung der Schadstoffkonzentration führen. Die Vorhersage kann alternativ oder ergänzend bereits solche automatisierten Maßnahmen vorsehen und/oder diese vorschlagen. Beispielsweise könnte als eine solche Maßnahme der Verkehr durch eine entsprechende Ampelschaltung umgeleitet werden und/oder Straßen vollständig gesperrt werden. Zudem könnten automatisiert und basierend auf der erfindungsgemäßen Vorhersage mehr Busse und/oder Straßenbahnen zur Verfügung gestellt werden.

Es ergeben sich zum erfindungsgemäßen Verfahren zum Erzeugen der Trainingsdaten beziehungsweise zum erfindungsgemäß trainierten neuronalen Netz gleichartige und gleichwertige Vorteile und Ausgestaltungen.

Gemäß einer vorteilhaften Ausgestaltung der Erfindung erfolgt die Veränderung $\Delta C$ des wenigstens einen Wertes $C_0$ der bereitgestellten Messreihe der Schadstoffkonzentrationen zusätzlich mittels eines verkehrsbedingten Anteils $\alpha$ an der Schadstoffkonzentration.

Mit anderen Worten wird der verkehrsbedingte Anteil an der Schadstoffkonzentration berücksichtigt. Typischerweise setzt sich die Schadstoffkonzentration eines Schadstoffes, beispielsweise von Stickoxid, aus mehreren Anteilen zusammen. Die Anteile sind hauptsächlich der Verkehr, die Gebäude und die Industrie sowie die Energieerzeugung. Der Anteil des Verkehrs, das heißt der verkehrsbedingte Anteil $\alpha$, ist typischerweise bekannt. Beispielsweise durch einen Vergleich mit einer weiteren nicht so stark durch den Verkehr belasteten Messstation. Dadurch kann vorteilhafterweise in effizienter Weise von den Schadstoffemissionen auf die Schadstoffimmission beziehungsweise Schadstoffkonzentrationen geschlossen werden, ohne dass eine explizite und aufwendige Berechnung oder Ermittlung erforderlich ist. Dieser approximative heuristische Ansatz ermöglicht somit eine effiziente Ermittlung der Schadstoffkonzentrationen aus den Schadstoffemissionen und somit zur Bereitstellung beziehungsweise Erzeugung des Trainingsdatensatzes.

In einer vorteilhaften Ausgestaltung der Erfindung erfolgt die Veränderung $\Delta C$ des wenigstens einen Wertes $C_0$ der bereitgestellten Messreihe der Schadstoffkonzentration mittels

$$\Delta C / C_0 = \alpha \Delta I / I_0.$$

Mit anderen Worten wird bevorzugt eine lineare Abhängigkeit zwischen der relativen Änderung der Schadstoffimmissionen und der relativen Änderung der Schadstoffkonzentrationen verwendet. Die relative Änderung der Schadstoffimmissionen wird gemäß der vorliegenden Erfindung durch das Transmissionsmodell ermittelt. Das heißt, dass ausgehend von einem Messwert der Messgröße, beispielsweise der Temperatur, der Windgeschwindigkeit und/oder der Windrichtung und/oder chemischen Stoffkonzentrationen, diese Messgröße in ihrem Wert verändert wird, und eine zum veränderten Messwert zugehörige neue Schadstoffimmission ermittelt und die relative Änderung zwischen der neuen Schadstoffimmission(zweite Schadstoffimmission) und der zum ursprünglichen Messwert der Messgröße zugehörigen Schadstoffimmission(erste Schadstoffimmission) berechnet wird. Die Schadstoffkonzentration, welche für das Trainieren des neuronalen Netzes erforderlich ist, wird mittels des verkehrsbedingten Anteils $\alpha$ aus der derart ermittelten relativen Änderung der Schadstoffimmissionen bestimmt. Dies wird insbesondere für jeden Wert beziehungsweise Zeitpunkt der ursprünglichen Messreihe der Schadstoffkonzentrationen durchgeführt. Mit anderen Worten wird jeder Wert $C_0$ der Messreihe der Schadstoffkonzentration um ein typischerweise verschiedenes $\Delta C$ verändert. Der Wert $C_1$ der derart neu gebildeten synthetischen Messreihe der Schadstoffkonzentration wird demnach für jeden Zeitpunkt $t$ durch $C_1(t) = C_0(t) + \Delta C(t)$ beziehungsweise für diskrete Zeitwerte $t_n$ durch $C_1(t_n) = C_0(t_n) + \Delta C(t_n)$ ermittelt. Es können ebenfalls nur Teilbereiche der Messreihe der Schadstoffkonzentration derart verändert werden, insbesondere lediglich ein Wert beziehungsweise Zeitpunkt der genannten Messreihe. Weitere mathematisch äquivalente Formulierungen und/oder Änderungen können vorgesehen sein.

Gemäß einer vorteilhaften Ausgestaltung der Erfindung wird ein verkehrsbedingter Anteil $\alpha$ im Bereich von 0,3 bis 0,5 verwendet.

Mit anderen Worten hat der Verkehr, welcher beispielsweise den Straßenverkehr umfasst, an der Schadstoffkonzentration, beispielsweise an einer Messstation an einer Straße, einen Anteil im Bereich von 0,3 bis 0,5. Besonders bevorzugt ist ein hoher lokaler verkehrsbedingter Anteil (Verkehrsanteil). Der verkehrsbedingte Anteil $\alpha$ ist grundsätzlich von den Umständen des Einzelfalls, beispielsweise der Stadt, der Straße, des Standortes der Messstation usw., abhängig. Dennoch hat sich gezeigt, dass hohe lokale verkehrsbedingte Anteile, bestenfalls in Kombination mit einem möglichst homogen urbanen Hintergrund, zur Ermittlung der relativen Änderung der Schadstoffkonzentration aus der relativen Änderung der Schadstoffimmissionen besonders gut geeignet ist.

In einer vorteilhaften Weiterbildung der Erfindung wird eine Stickoxidkonzentration als Schadstoffkonzentration und eine Stickoxidemission als Schadstoffemission verwendet.

Mit anderen Worten ist der betrachtete Schadstoff Stickstoffmonoxid und/oder Stickstoffdioxid (zusammenfassend $NO_x$). Weitere Stickoxidverbindungen können alternativ oder ergänzend vorgesehen sein. Ebenfalls können weitere Schadstoffe alternativ oder ergänzend vorgesehen sein. So kann die vorliegende Erfindung für eine Mehrzahl von Schadstoffen oder Schadstoffklassen verwendet werden. Insbesondere ebenfalls für Partikelklassen von Schadstoffen, beispielsweise $PM_{10}$ und/oder $PM_{2.5}$. Das Transmissionsmodell kann chemische Umwandlungsprozesse von Stickoxiden und/oder weiteren chemischen Stoffen umfassen. Insbesondere sind die auf eine Sonneneinstrahlung basierten chemischen Umwandlungsprozesse umfasst.

Gemäß einer vorteilhaften Ausgestaltung der Erfindung werden/wird als physikalische/technische Messgröße eine Temperatur, eine Windgeschwindigkeit und/oder eine Verkehrsstärke und/oder eine oder mehrere Stoffkonzentrationen verwendet.

Die Temperatur, die Windgeschwindigkeit und/oder die Verkehrsstärke und/oder chemische Stoffkonzentrationen sind technisch relevante Größen, insbesondere die Temperatur, Windrichtung/Windgeschwindigkeit und/oder die Sonneneinstrahlung und/oder die Verkehrsstärke, die die zeitliche und räumliche Verteilung und Ausbreitung der Schadstoffemission (Transmission) und somit die Bildung der Schadstoffkonzentration, insbesondere am Ort der Messstation, maßgeblich beeinflussen und/oder bestimmen. Mit anderen Worten hängt die Schadstoffkonzentration, die beispielsweise zu einem Zeitpunkt beziehungsweise innerhalb eines Zeitbereiches, durch eine Messstation gemessen wird, von der Temperatur, der Windgeschwindigkeit und/oder der Verkehrsstärke und/oder den chemischen Stoffkonzentrationen in der Luft und/oder der Sonneneinstrahlung (Watt pro Quadratmeter) ab. Grundsätzlich ist die Windgeschwindigkeit ein Vektorfeld, welches typischerweise eine bezüglich der Erdoberfläche horizontale und vertikale Komponente aufweist. Vorliegend können ebenfalls Teilgrößen der Windgeschwindigkeit, beispielsweise eine Windrichtung (horizontale Komponente), der Betrag der Windgeschwindigkeit und/oder eine Windstärke (Kategorisierung in Geschwindigkeitsintervalle), als Messgröße verwendet werden. Weitere physikalische/technische Messgrößen können alternativ oder ergänzend vorgesehen sein.

In einer vorteilhaften Ausgestaltung der Erfindung wurden die Messreihe der Schadstoffkonzentration und die Messreihe der Messgröße mittels einer Messstation innerhalb einer Stadt erfasst.

Das ist deshalb bevorzugt, da hohe Schadstoffkonzentrationen innerhalb von Städten auftreten und dort eine Vielzahl von Menschen direkt betroffen sind. Dort sind somit Maßnahmen zur Vermeidung solcher hohen Schadstoffkonzentrationen besonders erforderlich. Die vorliegende Erfindung und/oder eine ihrer Ausgestaltungen kann hierzu, durch eine verbesserte Vorhersage, die durch ein verbessert trainiertes neuronales Netz ermöglicht wird, einen entscheidenden Beitrag leisten.

Bevorzugt werden für das Verfahren gemäß der vorliegenden Erfindung und/oder einer ihrer Ausgestaltungen die genannten Messreihen erfasst.

Gemäß einer bevorzugten Ausgestaltung der Erfindung wird als Transmissionsmodell ein Modell verwendet, welches eine chemische Zusammensetzung der Luft und/oder chemische Reaktionen beziehungsweise Umwandlungsprozesse innerhalb der Luft berücksichtigt

Dadurch können vorteilhafterweise wichtige chemische Umwandlungsprozesse, die ebenfalls von der Temperatur, bestimmten Stoffkonzentrationen und/oder der Sonneneinstrahlung, insbesondere im UV und/oder optischen Bereich, abhängig sein können, berücksichtigt werden.

Weiterhin können die Schadstoffemissionen ebenfalls bevorzugt mittels eines Domain-Modells ermittelt werden.

Insbesondere umfasst das Domain-Modell die verkehrsspezifischen Schadstoffemissionen. Mit anderen Worten sind mittels des Domain-Modells die Schadstoffemissionen des Verkehrs, beispielsweise in einem Bereich einer Stadt und/oder an einer Straße, berechenbar. Das Domain-Modell modelliert somit die verkehrsspezifischen Schadstoffemissionen.

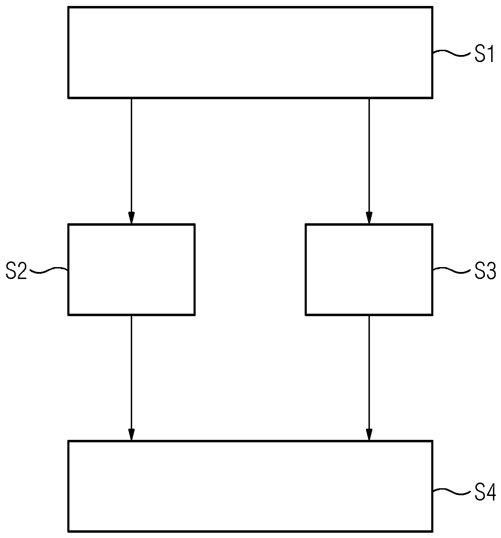

Weitere Vorteile, Merkmale und Einzelheiten der Erfindung ergeben sich aus den im Folgenden beschriebenen Ausführungsbeispielen sowie anhand der Zeichnung. Dabei zeigt die einzige Figur ein schematisiertes Ablaufdiagramm einer Ausgestaltung der Erfindung.

Gleichartige, gleichwertige oder gleichwirkende Elemente können in der Figur mit denselben Bezugszeichen versehen sein.

Die Figur zeigt ein Ablaufdiagramm beziehungsweise Flussdiagramm gemäß einer Ausgestaltung der vorliegenden Erfindung.

Zunächst wird in einem ersten Schritt S1 eine Messreihe für eine Schadstoffkonzentration $C_0(t)$, eine Temperatur $T_0(t)$, eine Windrichtung $W_0(t)$ und/oder eine Windgeschwindigkeit $v(t)$ bereitgestellt. Die Schadstoffkonzentration ist beispielsweise eine Stickoxidkonzentration. Die Schadstoffkonzentration und die Messgrößen, das heißt vorliegend die Temperatur, die Windrichtung und/oder die Windgeschwindigkeit wurden gemeinschaftlich erfasst. In diesem Sinne sind die Werte der Messgrößen den Werten der Schadstoffkonzentration zugeordnet. Dadurch werden für jeden Zeitpunkt, beispielsweise jede Stunde eines Tages, beispielsweise vier Werte bereitgestellt, nämlich die Schadstoffkonzentration für diesen Zeitpunkt, die Temperatur für diesen Zeitpunkt, die Windgeschwindigkeit für diesen Zeitpunkt und die Windgeschwindigkeit für diesen Zeitpunkt. Hierbei können durchschnittliche, gemittelte und/oder gewichtete Werte für den jeweiligen Zeitpunkt, beispielsweise über einen Zeitbereich von einer Stunde, verwendet werden. Weiterhin könnte entsprechend die Sonneneinstrahlung und/oder eine chemische Stoffkonzentration ermittelt werden.

Mit anderen Worten werden vorliegend exemplarisch vier Zeitreihen $C_0(t)$, $T_0(t)$, $W_0(t)$, $v(t)$ bereitgestellt, wobei für jeden Zeitpunkt der Zeitreihen, ein Messwert der Schadstoffkonzentration, ein Messwert der Temperatur, ein Messwert der Windgeschwindigkeit und ein Messwert der Windgeschwindigkeit vorliegt. Die Messwerte müssen nicht zu diesem Zeitpunkt erfasst worden sein, sondern können repräsentativ für diesen Zeitpunkt ausgewählt oder ermittelt worden sein, beispielsweise durch eine Mittelung. Beispielsweise umfassen die Zeitreihe 24 Werte, die zu den Stunden eines Tages korrespondieren.

In einem zweiten Schritt S2 wird mittels der gemessenen Zeitreihen $T_0(t)$, $W_0(t)$, $v(t)$ der Messgrößen und einer mittels eines Domain-Modells ermittelten Schadstoffemission eine erste Schadstoffimmission $I_0$ mittels eines Transmissionsmodells, für wenigstens einen der Zeitpunkte $t$, bevorzugt für alle Zeitpunkte $t$, berechnet. Die erste Schadstoffimmission $I_0$ basiert somit auf tatsächlichen Messwerten beziehungsweise Messdaten. Typischerweise sind hierbei Temperatur, Windrichtung und Windgeschwindigkeit relevant.

In einem dritten Schritt S3, der parallel zu S2 durchgeführt werden kann, wird wenigstens ein Wert wenigstens einer Messgröße verändert. Beispielsweise wird die zum Zeitpunkt $t$ vorliegende Temperatur um 3 Prozent erhöht, und dadurch eine neue synthetische Messreihe erzeugt. Die derart neu erzeugte Zeitreihe beziehungsweise Messreihe weist wenigstens einen Wert auf, der auf dieser Änderung basiert und demnach nicht gemessen wurde. In diesem Sinne ist die durch die Veränderung erzeugte Messreihe synthetisch. Anschließend wird aus den unveränderten Zeitreihen für die Windgeschwindigkeit und die Windrichtung und aus der veränderten Messreihe für die Temperatur eine zweite Schadstoffimmission $I_1$, für den Zeitpunkt an welchem der Messwert der Temperatur verändert wurde, mittels des Transmissionsmodells aus den Schadstoffemissionen, die unverändert bleiben, berechnet. Die zweite Schadstoffimmission $I_1$ basiert somit auf tatsächlichen Messwerten beziehungsweise Messdaten und der durch die Veränderung synthetisch erzeugten Messreihe.

Nach den Schritten S2 und S3 liegen somit für wenigstens einen Zeitpunkt zwei mittels des Transmissionsmodells ausgehend von der Schadstoffemission berechnete Schadstoffimmissionen $I_0$, $I_1$ vor.

In einem vierten Schritt S4 wird mittels der relativen Abweichung $\Delta I/I_0 = (I_1 - I_0)/I_0$ der berechneten Schadstoffimmission die relative Änderung der Schadstoffkonzentration mittels $\Delta C/C_0 = \alpha \Delta I/I_0$ berechnet. Hierbei ist mit $\alpha$ der verkehrsbedingte Anteil an der Schadstoffkonzentration bezeichnet. Beispielsweise weist $\alpha$ den Wert 0,4 auf.

Aus der relativen Änderung der Schadstoffkonzentration (an dem betrachteten Zeitpunkt) wird mittels der Messreihe der Schadstoffkonzentration eine neue synthetische Messreihe für die Schadstoffkonzentrationen dadurch erzeugt, dass der Messwert $C_0$, der zum betrachteten Zeitpunkt vorliegt, um $\Delta C$ verändert wird. Dadurch wird die neue Zeitreihe (synthetische Messreihe) erzeugt, die für das Trainieren des neuronalen Netzes zusätzlich zur ursprünglich bereitgestellten gemessenen Messreihe der Schadstoffkonzentrationen verwendet werden kann. Grundsätzlich kann das obenstehend beschriebene Vorgehen für alle Zeitpunkte oder Teile der Zeitpunkte durchgeführt werden.

Im Folgenden wird ein vereinfachtes Ausführungsbeispiel erläutert.

Für einen bestimmten Zeitpunkt und einem bestimmten Ort, beispielsweise dem Ort beziehungsweise Bereich der Messstation, liegt ein hoher Messwert der Stickoxidkonzentration, das heißt ein Messwert oberhalb des Schwellenwertes oder Grenzwertes, vor. Hierzu werden für diesen Zeitpunkt eine bestimmte Temperatur, Windrichtung und Windgeschwindigkeit gemessen.

Mittels des für die Schadstoffemissionen des Verkehrs spezifischen Domain-Modells wird für die gemessene Temperatur, Windrichtung und Windrichtung und/oder Verkehrsdichte/Verkehrsstärke (Eingabegrößen oder Eingabeparameter des Domain-Modells) die erste Schadstoffemission für diesen Zeitpunkt, beispielsweise 30 $\mu$g/m/s an Stickoxide, berechnet.

Basierend auf der berechneten Schadstoffemission wird mittels des Transmissionsmodells eine erste Schadstoffimmission an der Messstation ermittelt.

Anschließend wird eine weitere Berechnung mit einer leicht veränderten Temperatur, beispielsweise um 5 Prozent oder 5 Grad Celsius gegenüber der ursprünglich gemessenen Temperatur erhöht, mittels des Transmissionsmodells durchgeführt. Die Windgeschwindigkeit und die Windrichtung bleiben hierbei unverändert. Hieraus ergibt sich die zweite Schadstoffimmission, beispielsweise 33 µg/m/s an Stickoxide. Dadurch ergibt sich eine relative Änderung der Schadstoffimmission um 10 Prozent. Diese relative Änderung der Schadstoffimmission wird nun auf eine relative Änderung der Schadstoffkonzentration (an der Messstation) umgelegt beziehungsweise umgerechnet.

Die Schadstoffkonzentration umfasst typischerweise mehrere Anteile, beispielsweise einen Anteil des Verkehrs (verkehrsbedingter Anteil), einen Anteil der Gebäude und einen Anteil aus der Energieerzeugung. Beispielsweise ist der verkehrsbedingte Anteil $\alpha$ gleich 44 Prozent, der gebäudebedingte Anteil oder regionalbedingte Anteil 18 Prozent und der energieerzeugungsbedingte Anteil 38 Prozent. Insbesondere der verkehrsbedingte Anteil sinkt bezüglich Stickoxiden seit Jahren und wird sich voraussichtlich in den kommenden Jahren weiter reduzieren.

Aus dem verkehrsbedingten Anteilkann dann durch $\Delta C/C_0 = \alpha \Delta I/I_0$ auf die relative Änderung der Schadstoffkonzentration geschlossen werden. Bei einer relativen Änderung der Schadstoffimmission um 10 Prozent ergibt sich somit eine relative Änderung der Schadstoffkonzentration um 4,4 Prozent. Das heißt, dass sich die ursprünglich gemessene Schadstoffkonzentration zum vorliegend betrachteten Zeitpunkt um 4,4 Prozent ändern würde. Mit anderen Worten übersetzt sich eine 10 prozentige Änderung der Temperatur oder eine Änderung der Temperatur um 5 Grad Celsius in eine 4,4 prozentige Änderung der Schadstoffkonzentration.

Wird das obenstehend erläuterte Verfahren für jeden Zeitpunkt oder für weitere ausgewählte Zeitpunkte der gemessenen Zeitreihe für die Schadstoffkonzentrationen durchgeführt, so kann eine neue synthetische Zeitreihe beziehungsweise Messreihe für die Schadstoffkonzentration erzeugt werden. Das neuronale Netz kann dann mit dieser neu erzeugten Zeitreihe trainiert werden.

Das beschriebene Verfahren ist computergestützt und kann mit einem Rechner, einem zentralen oder dezentralen Server, in der Cloud oder mittels eines Quantencomputers durchgeführt werden. Weiterhin basiert das computergestützte Verfahren auf Messwerten von physikalischen Messgrößen, die als Eingangsgrößen beziehungsweise Eingangsparameter einbezogen sind.

Obwohl die Erfindung im Detail durch die bevorzugten Ausführungsbeispiele näher illustriert und beschrieben wurde, so ist die Erfindung nicht durch die offenbarten Beispiele eingeschränkt oder andere Variationen können vom Fachmann hieraus abgeleitet werden, ohne den Schutzumfang der Erfindung zu verlassen.

Bezugszeichenliste

| S1 | erster Schritt |
| S2 | zweiter Schritt |
| S3 | dritter Schritt |
| S4 | vierter Schritt |

## Patentansprüche

1. Computergestütztes Verfahren zum Erzeugen von Trainingsdaten für ein neuronales Netz, wobei das neuronale Netz zum Ermitteln einer Schadstoffkonzentration an einer Messstation aus wenigstens einer Schadstoffemission ausgebildet ist, **gekennzeichnet durch** folgende Schritte:

    - Bereitstellen wenigstens einer Messreihe der Schadstoffkonzentration mit wenigstens einem Messwert, der oberhalb eines festgelegten Schwellenwertes ist;
    - Bereitstellen wenigstens einer Messreihe für eine zur gemessenen Schadstoffkonzentration zugehörige physikalische Messgröße, die eine Temperatur, eine Windgeschwindigkeit und/oder eine Windrichtung, eine Verkehrsstärke, und/oder eine chemischen Stoffkonzentrationen ist, wobei die gemessene Schadstoffkonzentration von der Messgröße abhängig ist;
    - Bereitstellen eines Transmissionsmodells, wobei das Transmissionsmodell einen Zusammenhang zwischen der Schadstoffemission, der Messgröße und der Schadstoffimmission an der Messstation modelliert;
    - Berechnen eines ersten Wertes $I_0$ der Schadstoffimmission an der Messtation mittels des Transmissionsmodells aus der Schadstoffemission, wobei hierzu wenigstens ein zu einem Wert $C_0$ der bereitgestellten gemessenen Schadstoffkonzentration zugehöriger Messwert der Messgröße verwendet wird;
    - Berechnen eines zweiten Wertes $I_1$ der Schadstoffimmission an der Messstation mittels des Transmissionsmodells aus der Schadstoffemission, wobei hierzu der für das Berechnen des ersten Wertes $I_0$ der Schadstoffimmission verwendete Messwert der Messgröße numerisch verändert wird; und
    - Erzeugen einer synthetischen Messreihe als Trainingsdaten durch eine Veränderung $\Delta C$ des Wertes $C_0$ der bereitgestellten gemessenen Messreihe der Schadstoffkonzentrationen, wobei die Veränderung $\Delta C$ des wenigstens einen Wertes $C_0$ der bereitgestellten Messreihe der Schadstoffkonzentration mittels $\Delta C/C_0 = \alpha(I_1 - I_0)/I_0$ erfolgt, wobei $\alpha$ ein verkehrsbedingter Anteil an der Schadstoffkonzentration ist.

2. Computergestütztes Verfahren gemäß Anspruch 1, **gekennzeichnet dadurch, dass** ein verkehrsbedingter Anteil $\alpha$ im Bereich von 0,3 bis 0,5 verwendet wird.

3. Computergestütztes Verfahren gemäß einem der vorhergehenden Ansprüche, **gekennzeichnet dadurch, dass** eine Stickoxidkonzentration als Schadstoffkonzentration und eine Stickoxidemission als Schadstoffemission verwendet wird.

4. Computergestütztes Verfahren gemäß einem der vorhergehenden Ansprüche, **gekennzeichnet dadurch, dass** die Messreihe der Schadstoffkonzentration und die Messreihe der Messgröße mittels einer Messstation innerhalb einer Stadt erfasst wurden.

5. Computergestütztes Verfahren gemäß einem der vorhergehenden Ansprüche, **gekennzeichnet dadurch, dass** als Transmissionsmodell ein Modell verwendet wird, welches eine chemische Zusammensetzung der Luft und/oder chemischen Reaktionen innerhalb der Luft berücksichtigt.

6. Computergestütztes Verfahren zum Trainieren eines neuronalen Netzes, wobei das neuronale Netz zum Ermitteln einer Schadstoffkonzentration aus wenigstens einer Schadstoffemission ausgebildet ist, **gekennzeichnet dadurch, dass** zum Trainieren des neuronalen Netzes ein gemäß einem der vorhergehenden Ansprüche erzeugter Trainingsdatensatz verwendet wird.

7. Computergestütztes Verfahren zum Ermitteln einer Schadstoffkonzentration mittels eines neuronalen Netzes und mittels eines Domain-Modells der Schadstoffemission, wobei das neuronale Netz zum Ermitteln einer Schadstoffkonzentration aus der Schadstoffemission ausgebildet und gemäß Anspruch 6 trainiert ist, wobei das Domain-Modell einen Zusammenhang zwischen einer physikalischen Messgröße, insbesondere einer Temperatur, einer Windgeschwindigkeit und/oder einer Verkehrsstärke, und der Schadstoffemission modelliert, **gekennzeichnet durch** folgende Schritte:

- Berechnen eines Wertes der Schadstoffemission mittels des Domain-Modells, wobei hierzu wenigstens ein Messwert der Messgröße verwendet wird; und
- Ermitteln der Schadstoffkonzentration aus dem berechneten Wert der Schadstoffemission mittels des neuronalen Netzes.

**Claims**

1. Computer-aided method for generating training data for a neural network, wherein the neural network is designed to determine a pollutant concentration at a measurement station from at least one pollutant emission, **characterized by** the following steps:

- providing at least one measurement series of the pollutant concentration containing at least one measured value that is above a defined threshold value;
- providing at least one measurement series for a physical measured variable related to the measured pollutant concentration, which is a temperature, a wind speed and/or a wind direction, a traffic level, and/or chemical substance concentrations, the measured pollutant concentration being dependent on the measured variable;
- providing a transmission model, wherein the transmission model models a relationship between the pollutant emission, the measured variable and the pollutant immission at the measurement station;
- computing a first value $I_0$ of the pollutant immission at the measurement station by means of the transmission model from the pollutant emission, this being accomplished by using at least one measured value of the measured variable that is related to a value $C_0$ of the provided measured pollutant concentration;
- computing a second value $I_1$ of the pollutant immission at the measurement station by means of the transmission model from the pollutant emission, this being accomplished by numerically altering the measured value of the measured variable that is used for computing the first value $I_0$ of the pollutant immission; and
- generating a synthetic measurement series as training data by means of an alteration $\Delta C$ of the value $C_0$ of the provided measured measurement series of the pollutant concentrations, the alteration $\Delta C$ of the at least one value $C_0$ of the provided measurement series of the pollutant concentration being made by means of $\Delta C/C_0 = \alpha(I_1 - I_0)/I_0$, where $\alpha$ is a traffic-related proportion of the pollutant concentration.

2. Computer-aided method according to Claim 1, **characterized in that** a traffic-related proportion $\alpha$ in the range from 0.3 to 0.5 is used.

3. Computer-aided method according to either of the preceding claims, **characterized in that** a nitrogen oxide concentration is used as pollutant concentration and a nitrogen oxide emission is used as pollutant emission.

4. Computer-aided method according to one of the preceding claims, **characterized in that** the measurement series of the pollutant concentration and the measurement series of the measured variable were captured by means of a measurement station within a town.

5. Computer-aided method according to one of the preceding claims, **characterized in that** the transmission model used is a model which takes into account a chemical composition of the air and/or chemical reactions within the air.

6. Computer-aided method for training a neural network, wherein the neural network is designed to determine a pollutant concentration from at least one pollutant emission, **characterized in that** a training dataset generated according to one of the preceding claims is used to train the neural network.

7. Computer-aided method for determining a pollutant concentration by means of a neural network and by means of a domain model of the pollutant emission, wherein the neural network is designed to determine a pollutant concentration from the pollutant emission and is trained according to Claim 6, wherein the domain model models a relationship between a physical measured variable, in particular a temperature, a wind speed and/or a traffic level, and the pollutant emission, **characterized by** the following steps:

- computing a value of the pollutant emission by means of the domain model, this being accomplished by using at least one measured value of the measured variable; and
- determining the pollutant concentration from the computed value of the pollutant emission by means of the neural network.

**Revendications**

1. Procédé assisté par ordinateur de production de données d'entraînement d'un réseau neuronal, dans lequel le réseau neuronal est constitué pour la détermination d'une concentration de substance polluante en un poste de mesure d'au moins une émission de substance polluante, **caractérisé par** les stades suivants :

- se procurer au moins une série de mesures de la concentration de substance polluante ayant au moins une valeur de mesure, qui est au-dessus d'une valeur de seuil fixée ;
- se procurer au moins une série de mesures d'une grandeur de mesure physique appartenant à la concentration de substance polluante mesurée, qui est une température, une vitesse du vent et/ou une direction du vent, une intensité du trafic et/ou une concentration de substance chimique, dans lequel la concentration de substance polluante mesurée dépend de la grandeur de mesure ;
- se procurer un modèle de transmission, dans lequel le modèle de transmission modélise une relation entre l'émission de substance polluante, la grandeur de mesure et l'émission de substance polluante au poste de mesure ;
- calculer une première valeur $I_0$ de l'émission de substance polluante au poste de mesure au moyen du modèle de transmission à partir de l'émission de substance polluante, dans lequel on utilise à cet effet au moins une valeur de mesure, appartenant à une valeur $C_0$ de la concentration de substance polluante mesurée que l'on s'est procurée, de la grandeur de mesure ;
- calcul d'une deuxième valeur $I_1$ de l'émission de substance polluante au poste de mesure au moyen du modèle de transmission à partir de l'émission de substance polluante, dans lequel on modifie numériquement à cet effet la valeur de mesure, utilisée pour le calcul de la première valeur $I_0$ de l'émission de substance polluante, de la grandeur de mesure ; et
- production d'une série de mesures synthétiques comme données d'entraînement par une modification $\Delta C$ de la valeur $C_0$ de la série de mesures mesurée que l'on s'est procurée, des concentrations de substance polluante, dans lequel la modification $\Delta C$ de la au moins une valeur $C_0$ de la série de mesures, que l'on s'est procurée, de la concentration de substance polluante s'effectue au moyen de $\Delta C/C_0 = \alpha(I_1-I_0)/I_0$, $\alpha$ étant une proportion due au trafic de la concentration de substance polluante.

2. Procédé assisté par ordinateur suivant la revendication 1, **caractérisé en ce que** l'on utilise une proportion $\alpha$ due au trafic dans la plage de 0,3 à 0,5.

3. Procédé assisté par ordinateur suivant l'une des revendications précédentes, **caractérisé en ce que** l'on utilise une concentration d'oxyde d'azote comme concentration de substance polluante et une émission d'oxyde d'azote comme émission de substance polluante.

4. Procédé assisté par ordinateur suivant l'une des revendications précédentes, **caractérisé en ce que** l'on a relevé dans une ville au moyen d'un poste de mesure la série de mesures de la concentration de substance polluante et la série de mesures de la grandeur de mesure.

5. Procédé assisté par ordinateur suivant l'une des revendications précédentes, **caractérisé en ce que** l'on utilise comme modèle de transmission, un modèle, qui tient compte d'une composition chimique de l'air et/ou de réactions chimiques dans l'air.

**6.** Procédé assisté par ordinateur d'entraînement d'un réseau neuronal, dans lequel le réseau neuronal est constitué pour la détermination d'une concentration de substance polluante, à partir d'au moins une émission de substance polluante, **caractérisé par le fait que**, pour l'entraînement du réseau neuronal, on utilise un ensemble de données d'entraînement produit suivant l'une des revendications précédentes.

**7.** Procédé assisté par ordinateur de détermination d'une concentration de substance polluante au moyen d'un réseau neuronal et au moyen d'un modèle de domaine de l'émission de substance polluante, dans lequel le réseau neuronal est constitué pour la détermination d'une concentration de substance polluante à partir de l'émission de substance polluante et est entraîné suivant la revendication 6, dans lequel le modèle de domaine modélise une relation entre une grandeur de mesure physique, en particulier une température, une vitesse du vent et/ou une intensité du trafic, et l'émission de substance polluante, **caractérisé par** les stades suivants :

- calcul d'une valeur de l'émission de substance polluante au moyen du modèle de domaine, dans lequel on utilise à cet effet au moins une valeur de mesure de la grandeur de mesure ; et
- détermination de la concentration de substance polluante à partir de la valeur calculée de l'émission de substance polluante au moyen du réseau neuronal.

S1

S2

S3

S4

EP 4 107 522 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **ZHENG, YU et al.** *Forecasting Fine-Grained Air Quality Based on Big Data*, 10 August 2015, 2267-2276